# NEUE EUROPÄISCHE PATENTSCHRIFT

(11) **EP 2 210 576 B2**
(45) Veröffentlichungstag und Bekanntmachung des Hinweises auf die Entscheidung über den Einspruch: **17.08.2016**
(45) Hinweis auf die Patenterteilung: 11.07.2012
(21) Anmeldenummer: 10151380.2
(22) Anmeldetag: 22.01.2010
(51) Int. Cl.: A61F 5/30, A61F 5/03, A61F 5/32, A61F 13/14

(54) **Thorax-Bandage mit mindestens einer Pelotte**
Thorax bandage with at least one pelotte
Bandage de thorax doté d'au moins une pelote

(30) Priorität: 22.01.2009 DE 102009000374
(43) Veröffentlichungstag der Anmeldung: 28.07.2010
(73) Patentinhaber: Keuchen, Karsten, 52078 Aachen (DE)
(72) Erfinder: Keuchen, Karsten, 52078, Aachen (DE); Buchmann, Bodo, 52134, Herzogenrath (DE); Keuchen, Karsten, 52078, Aachen (DE)
(74) Vertreter: Bauer, Dirk

(56) Entgegenhaltungen:
- WO-A1-90/05502
- WO-A2-2005/107659
- WO-A2-2007/047424
- WO-A2-2007/048162
- AT-A1- 502 986
- FR-A1- 2 670 668
- JP-A- 2002 345 868
- US-A- 4 245 628
- US-A1- 2006 229 538

## Beschreibung

Die Erfindung betrifft eine Thorax-Bandage mit einem mindestens eine Pelotte aus einem elastischen Material, insbesondere einem Schaumstoff, aufweisenden Mittelteil an dessen gegenüberliegenden horizontalen Enden jeweils die Enden eines zirkulären Rückengurts angeschlossen sind und an dessen oberem Ende beabstandet voneinanderdie Enden zweier Schultergurte angeschlossen sind, wobei beide Schultergurte im Rückenbereich eines Patienten mit dem Rückgurt verbunden sind.

Eine derartige Thorax-Bandage kommt nach operativen Eingriffen im Thoraxbereich, wie beispielsweise nach einer Herz- oder Lungenoperation, zum Einsatz, um die Wundheilung des im Bereich des Sternums (Brustbein) verlaufenden Narbenschnitts zu verbessern. Insbesondere dient die Thorax-Bandage dazu, auf die infolge der Operation aufgeweiteten Brustkorbhälften Druck auszuüben und sie in ihre ursprüngliche Lage zu pressen, so dass das Gewebe und die Haut im Bereich des Narbenschnitts nicht auseinander klafft und die sich gegenüberliegenden Bereiche des Narbenschnitts nicht unter zu starker Zugspannung stehen und somit besser und schneller verheilen beziehungsweise zusammenwachsen können.

### Stand der Technik

Allgemein bekannte Thorax-Bandagen weisen typischerweise zwei getrennte, mittels eines flexiblen Bandes miteinanderverbundene Pelotten auf, diejeweils auf eine Seite des Narbenschnitts im Bereich links und rechts neben dem Sternum angeordnet werden. Zum einen werden die Pelotten von einem umlaufenden Rükkengurt und zum anderen von zwei Schultergurten, die jeweils an einer Pelotte und auf der gegenüberliegenden Seite an dem Rückengurt angreifen, in ihrer Position gehalten.

Nachteil der bekannten Thorax-Bandagen ist, dass die neben dem Narbenschnitt angeordneten Pelotten infolge derauf sie durch den Rückengurt beziehungsweise die Schultergurte wirkenden Kräfte leicht verkippen können, so dass der gewünschte Effekt der Thorax-Bandage hinsichtlich der Lage und der Richtung der auf den Brustkorb wirkenden Kraft negativ beeinflusst wird. Eine optimale Wirkung einer Thorax-Bandage stellt sich nämlich ein, wenn die auf den Thorax wirkenden Kräfte möglichst senkrecht zur Körperoberfläche verlaufen und seitlich des Narbenschnitts angreifen.

Aus der DE 20 2004 001381 U1 ist darüber hinaus eine postoperative Compressions-Bandage bekannt, die speziell für Frauen bestimmt ist. Es handelt sich hierbei um eine Bandage, die im Bereich des Sternums ein flexibles Häckchen-Ösen-Band besitzt, dass zum Sternum hin mit einer Polsterung nach Art einer Pelotte unterlegt ist. Mit der bekannten Bandage können lediglich Umfangskräfte und insbesondere keine auf das Körperinnere gerichtete radiale Kräfte übertragen werden. Problematisch ist bei der bekannten Bandage insbesondere der Umstand, dass beidseitig des Sternums im Bereich der Rippen eingearbeitete Stäbchen, die die Bandage stabilisieren sollen. Im praktischen Einsatz kann es jedoch insbesondere bei Frauen mit starken Brüsten zu einem Einquetschen derselben kommen, wodurch der Tragekomfort stark eingeschränkt ist.

Ferner offenbart die WO 2007/048162 A2 eine Stützvorrichtungfürden Brustkorb mitmehren den Brustkorb umschließenden gurtartigen Zugelementen und Auflageelementen in Form von Pelotten, die mit Hilfe der Zugelemente gegen den Brustkorb angedrückt werden. Die aus einem weichen Schaumstoff bestehenden Auflageelemente, d.h. Pelotten, sind in Stofftaschen angeordnet, an die die Zugelemente angeschlossen sind. Zwischen den beiden benachbarten Auflageelementen befindet sich ein Abschnitt der Zugmittel mit geringerer Elastizität als im übrigen, d.h. insbesondere Seiten- und Rückenbereich, der Zugelemente. Nachteilig bei der bekannten Stützvorrichtung ist der Umstand, dass bei einer Krafteinleitung in die Stofftaschen sich sowohl diese als auch die darin befindlichen Schaumstoffpelotten in ungewünschter Form verwinden und verformen. Dies kann auch durch die Mehrzahl von Zugmitteln zwischen den beiden Pelotten nicht verhindert werden.

Schließlich beschreibt die AT 502 986 A1 noch eine Stützbandage für Wunden im Brustkorbbereich, die für Wunden nach Lungenoperationen verwendet werden soll und ein den Brustkorb umschließendes Stützband aufweist, das eine im Bereich der zu stabilisierenden Wunde befindliche Aussparung besitzt. Entlang eines Teils des Randes der Aussparung in dem Stützband befindet sich ein Polsterelement. Die vorgenannte Stützbandage ist aufgrund ihrer Konzipierung für Lungenoperationen, bei denen der Brustkorb seitlich hinten eröffnet wird, nicht dazu geeignet, den Thorax im Bereich des Sternums, z.B. nach einer Herzoperation, adäquat zu stützen.

### Aufgabe

Unter Berücksichtigung des vorstehend beschriebenen Standes der Technik liegt der Erfindung die Aufgabe zugrunde, eine Thorax-Bandage bereit zu stellen, die sich durch eine optimale Krafteinwirkung auf den Thorax auszeichnet.

### Lösung

Erfindungsgemäß wird diese Aufgabe dadurch gelöst, dass das Mittelteil eine Trägerplatte aufweist, an der die mindestens eine Pelotte befestigt ist, die aus einem im Vergleich mit dem Material der Pelotte wesentlich steiferen Material besteht, und an die der Rückengurt und die Schultergurte angeschlossen sind. Darüber hinaus überbrückt die Trägerplatte den Bereich des Sternums starr, wobei die mindestens eine Pelotte zwei lang gestreckte, parallel zu dem Sternum des Patienten verlaufende und seitlich neben diesem am Thorax anliegende Druckbereiche aufweist, die von einer rinnenförmigen und parallel zu den Druckbereichen verlaufenden Vertiefung im Bereich des Sternum voneinander getrennt sind.

Mit Hilfe der Trägerplatte wird die mindestens eine Pelotte demnach in ihrer Lage fixiert und neigt nicht mehrdazu, aufgrund der auf sie wirkenden Kräfte zu verkippen. Die Kräfte, die über den Rückengurt und die Schultergurte auf die Pelotte wirken, greifen erfindungsgemäß nicht an der Pelotte selbst an, sondern an der Trägerplatte mit wesentlich steiferen Eigenschaften. Die Trägerplatte besitzt im Vergleich mit der polsternden Pelotte eine wesentlich größere Biegesteifigkeit, so dass auch mit tangentialer Komponente über die Gurte an der Trägerplatte angreifende Kräfte im Wege der Kräftezerlegung mit ihrer radial zur Körpermittelachse gerichteten Komponente die gewünschte Stabilisierung der Rippenenden seitlich neben dem Sternum erreichen können.

Ein weiterer Vorteil der Erfindung besteht darin, dass die durch die Pelotte auf den Brustkorb eines Patienten wirkenden Kräfte senkrecht auf diesen wirken, wodurch eine optimale Wirkung derThorax-Bandage erzielt wird und eine optimale Stabilisation des Brustkorbs erreicht ist. Darüber hinaus kann ein Verwerfen oder Verwinden der Pelotte nicht mehr auftreten, so dass insgesamt größere Kräfte zielgerichtet auf die betreffenden Thoraxregionen ausgeübt werden können.

Eine besonders stabile Lage der mindestens einen Pelottewird dadurch erreicht, dass die Trägerplatte den Bereich des Sternums eines Patienten starr überbrückt, da die Trägerplatte auf diese Weise aufgrund ihrer Abmessungen eine hohe Steifigkeit erhält.

Dadurch, dass die mindestens eine Pelotte zwei lang gestreckte, parallel zu dem Sternum des Patienten verlaufende und seitlich neben diesem am Thorax anliegende Druckbereiche aufweist, die von einer rinnenförmigen und parallel zu den Druckbereichen verlaufenden Vertiefung im Bereich des Sternum voneinander getrennt sind, liegt der Narbenschnitt in jedem Fall frei und die Pelotte kommt dort nicht zur Anlage an dem Körper eines Patienten. Die Luftzirkulation im Bereich des Narbenschnitts wird nicht beeinträchtigt und es ist eine optische Kontrolle der Narbe möglich, ohne die Thorax-Bandage ausziehen zu müssen.

Alternativ kann die erfindungsgemäße Thorax-Bandage auch zwei lang gestreckte Pelotten aufweisen, die parallel zu dem Sternum des Patienten verlaufen und seitlich neben diesem am Thorax des Patienten anliegen. Dies istvon Vorteil, dafürdie Herstellung einer individuell an einen Körper angepassten Thorax-Bandage standardisierte Pelotten eingesetzt werden können, die lediglich hinsichtlich ihrer Position auf dem Körper des Patienten auf der Trägerplatte variiert werden müssen.

Eine Weiterentwicklung der erfindungsgemäßen Thorax-Bandage sieht einen langgestreckten Freiraum zwischen den beiden Pelotten bzw. den beiden Druckbereichen einer Pelotte vor, wobei der Freiraum im Querschnitt eine Breite zwischen 3 cm und 7 cm und eine Höhe zwischen 2 cm und 5 cm aufweist.

Wenn die Trägerplatte im Bereich des Freiraums zwischen zwei Pelotten mit mindestens einem Durchbruch, vorzugsweise mit einer Reihe hintereinander angeordneter Durchbrüche versehen ist, lässt sich eine Operationswunde im Bereich des Sternums durch den Durchbruch bzw. die Reihe der Durchbrüche hindurch visuell kontrollieren, ohne dass es erforderlich wäre, hierzu die gesamte Thorax-Bandage zu entfernen, was für den Patienten kurz nach einer Operation mit größeren Unannehmlichkeiten verbunden sein kann.

Vorteilhafterweise ist die mindestens eine Pelotte lösbar mit der Trägerplatte verbunden, wobei sich hiezu insbesondere mindestens ein einstückig mit der Pelotte verbundener Verbindungszapfen eignet, der formschlüssig und/oder reibschlüssig in einen angepassten Durchbruch in der Trägerplatte eingreift. Eine derart lösbare Pelotte ist im Bedarfsfall, beispielsweise infolge starker Verschmutzung, leicht auszutauschen, ohne den Wechsel einer gesamten Thorax-Bandage zu bedingen. Die Trägerplatte kann demgegenüber mehrfach wiederverwendetwerden, insbesondere wenn sie aus leicht abwaschbarem Material gefertigt ist.

Eine besonders einfache und flexible Art der Verbindung zwischen dem Rückengurt beziehungsweise den Schultergurten und der Trägerplatte wird dadurch erreicht, dass die Enden des Rückengurtes und/oder der Schultergurte in Form von Schlaufen durch randnah angeordnete Schlitze in der Trägerplatte geführt sind. Ferner zeichnet sich eine derartige Verbindung durch eine hohe Stabilität und Haltbarkeit aus, da die Krafteinleitung in dieTrägerplatte über verhältnismäßig große Bereiche verteilt wird, nämlich jeweils über die gesamte Länge einer Wandung der Schlitze, wobei die jeweiligen Gurte im Bereich der Schlitze doppelt liegen. Die Verbindung der Gurte mit der Trägerplatte über Schlaufen ist des Weiteren von Vorteil, da eine Anpassung der Gurtlängen an die individuellen Erfordernisse eines Patienten leicht und ohne großen Aufwand vorgenommen werden kann, indem die Länge der die Schlaufe bildenden überlappenden Bereiche der Gurte variiert wird.

Alternativ zu Schlitzen in der Trägerplatte können an letzterer auch Ösen, insbesondere solche mit einer flach rechteckförmigen Öffnung befestigt sein, um Schlaufen von Gurten hindurch zu führen.

Dabei ist es ferner vorteilhaft, wenn der Rükkengurt und/oder die Schultergurte mit mindestens einem Abschnitt aus einem in ihre Längsrichtung elastisch dehnbarem Material versehen sind, da auf diese Weise zwar ein ausreichender Druck auf den Brustkorb ausgeübt wird, jedoch auch eine gewisse Flexibilität erhalten bleibt, die den Tragekomfort der Thorax-Bandage deutlich erhöht.

Darüber hinaus ist es von Vorteil, wenn die Schlaufen des Rückengurts und/oder der Schultergurte mit Klettverschlüssen versehen sind, die eine Längeneinstellung des Rückengurtes und/oder der Schultergurte erlauben. Somit ist eine Anpassung der Thorax-Bandage an die Bedürfnisse eines Patienten weiter vereinfacht. Auch können die Klettverschlüsse gleichzeitig als Verschlüsse der Thorax-Bandage dienen, die ein An- und Ausziehen derselben besonders angenehm machen, insbesondere da die Beweglichkeit eines frisch operierten Patienten stark eingeschränkt ist.

Alternativ zu Klettverschlüssen ist es auch möglich, die wirksame Länge des Rückengurts und/oder der Schultergurte jeweils mittels eines Rasterverschlusses einstellbar zu gestalten. Dabei sind mit widerhakenförmigen Zähnen versehene Zungen an den Enden des Rückengurts und/oder der Schultergurte rastend in einer Vielzahl von Positionen in zugeordneten Schließköpfen arretierbar, die an der Trägerplatte befestigt, beispielsweise damit vernietet, sind. Die insbesondere aus dem Sportsektor (Ski- und Snowboard-Bindungen sowie Helmverschlüsse) bekannten Rasterverschlüsse zeichnen sich durch eine sehr einfache Bedienung, eine hohe Lebensdauersowie hinreichend hohe Schließkräfte aus. In Bezug auf die erfindungsgemäße Thorax-Bandage begünstigen die freien Zungen an den Enden der Gurte die Ausführung des Schließvorgangs durch den Patienten selbst, ohne dass dieser größere Schulter- oderArmbewegungen ausführen muss. In einerfür den Patienten nach einer Herzoperation sehr angenehmen Stellung mit gekreuzten Armen vor dem Brustkorb lassen sich die Zungen in die Schließköpfe der Trägerplatte einziehen und einzeln nacheinander/eventuell in mehreren Schritten durch einfaches Ziehen an den Zungenenden solange spannen, bis der erforderliche Anpressdruck im Bereich der Rippenenden erreicht ist. DerÖffnungsvorgang gestaltet sich ebenfalls sehr einfach, indem die federnd gelagerten Sperrklinken der Schließköpfe von den Verzahnungen derZungen abgehoben werden, wodurch der Verschluss lösbar ist.

Um den Tragekomfort der erfindungsgemäßen Thorax-Bandage weiter zu steigern, ist es vorgesehen, dass derzirkuläre Rückengurt im Bereich der Achsel des Patienten mit einem Ausschnitt und einer entlang des Randes des Ausschnitts verlaufenden Polsterung aus einem elastischen Material versehen ist.

Ferner ist es weiterhin vorteilhaft, wenn als Material für die Trägerplatte Polyethylen oder Polypropylen vorgesehen ist, wobei die Trägerplatte als Kunststoff-Tiefziehteil mit einer Dicke zwischen ca. 2 mm und 5 mm hergestellt ist. Eine derartige Trägerplatte lässt sich leicht reinigen und desinfizieren.

Gemäß einerweiteren vorteilhaften Ausgestaltung der erfindungsgemäßen Thorax-Bandage ist die Trägerplatte mit an ihrer dem Hals eines Patienten zugewandten Oberseite mit zwei armförmigen Fortsätzen versehen, die sich jeweils in Richtung auf eine Schulter des Patienten zu erstrecken. Hierdurch erhält die Trägerplatte eine insgesamt Y-förmige Gestalt. Die Schultergurte sollten bei einer derartigen Ausgestaltung vorzugsweise an die freien Enden der Fortsätze anschließen.

Schließlich ist nach der Erfindung noch vorgesehen, dass der Rückengurt und die Schultergurte von einem auf dem Rücken des Patienten angeordneten flächigen Rückenteil ausgehen, das vorzugsweise eine Breite von mindestens 20 cm und eine Länge von mindestens 40 cm besitzt, wobei der Rückengurt an die Unterseite des Rückenteils und die Schultergurte an die Oberseite des Rückenteils anschließen. Auf diese Weise wird ein besonders hoherTragekomfort erzielt, da Druckstellen beim Liegen auf dem Rücken sicher vermieden werden.

### Ausführungsbeispiel

Die Erfindung wird nachfolgend anhand mehrerer Ausführungsbeispiele erfindungsgemäßer Thorax-Bandagen, die in den Figuren dargestellt sind, näher erläutert.

Es zeigt
- Fig. 1:: eine Ansicht eines Brustbereichs eines Patienten mit einer Thorax-Bandage in einer ersten Ausführungsform,
- Fig. 2:: eine Seitenansicht des Patienten aus Figur 1,
- Fig. 3:: eine Rückansicht des Patienten aus Figur 1,
- Fig. 4 und 5:: jeweils eine dreidimensionale Ansicht der Trägerplatte aus Figur 1,
- Fig. 6:: eine Draufsicht auf die Rückseite der Trägerplatte mit Pelotten aus Figur 1,
- Fig. 7:: eine Draufsicht auf den Rückengurt aus Figur 1,
- Fig. 8:: eine Ansicht eines Brustbereichs eines Patienten mit einer Thorax-Bandage in einer zweiten Ausführungsform,
- Fig. 9:: eine Seitenansicht des Patienten aus Figur 8,
- Fig. 10:: eine Rückansicht des Patienten aus Figur 8,
- Fig. 11:: eine Seitenansicht der Trägerplatte aus Figur 8,
- Fig. 12:: eine Draufsicht auf die Rückseite der Trägerplatte mit Pelotten aus Figur 8,
- Fig. 13:: eine Draufsicht auf die Vorderseite der Trägerplatte aus Figur 8
- Fig. 14:: eine Seitenansicht einer Pelotte aus den Figuren 11 bis 13,
- Fig. 15:: eine Ansicht eines Brustbereichs eines Patienten mit einer Thorax-Bandage in einer dritten Ausführungsform,
- Fig. 16:: eine Seitenansicht des Patienten aus Figur 15,
- Fig. 17:: eine Rückansicht des Patienten aus Figur 15,
- Fig. 18:: eine Draufsicht auf die Vorderseite der Trägerplatte der dritten Ausführungsform der Thorax-Bandage,
- Fig. 19:: eine Draufsicht auf die Rückseite der Trägerplatte gemäß Figur 18 und
- Fig. 20:: eine schräge Seitenansicht auf die Trägerplatte gemäß Figur 18.

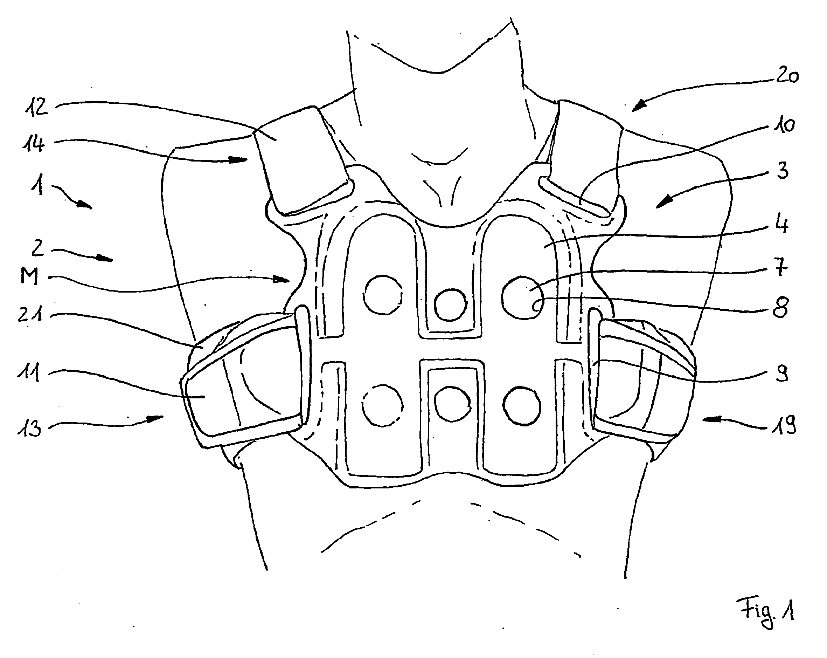

In der Figur 1 ist der Brustbereich 1 eines Patienten gezeigt, dereine erfindungsgemäßeThorax-Bandage 2 trägt. Die Thorax-Bandage 2 besteht aus einer ein Mittelteil M bildenden Trägerplatte 3, die sich über einen Großteil des Brustbereichs 1 erstreckt und über zwei aus derZeicheneben heraustretende erhabene Bereiche 4 verfügt, die jeweils eine Mulde 5 für in der Figur 1 nicht sichtbare Pelotten 6 bilden. Die zwei Pelotten 6 sind lang gestreckt und verlaufen jeweils rechts und links parallel zu dem Sternum des Patienten, in dessen Bereich sich ein Narbenschnitt befindet. Die Pelotten 6 verfügen jeweils über zwei integrale Verbindungszapfen 7, mit denen sie formschlüssig in jeweils zwei in der Mulde 5 der Trägerplatte 3 vorgesehene Durchbrüche 8 eingreifen.

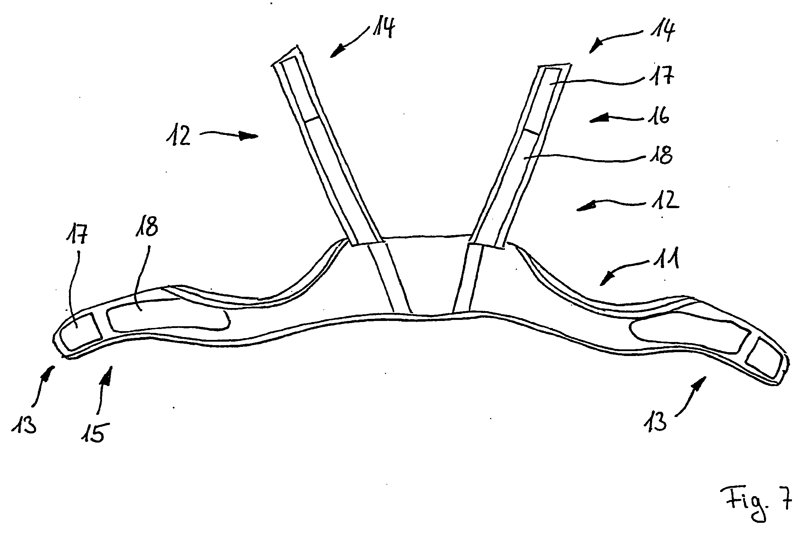

Die Trägerplatte 3 ist in ihren Randbereichen mit vier Schlitzen 9, 10 versehen, wobei durch die beiden unteren Schlitze 9 ein Rückengurt 11 und durch die beiden oberen Schlitze 10 jeweils ein Schultergurt 12 geführt ist. Der Rückengurt 11 und die beiden Schultergurte 12 weisen jeweils an ihren Enden 13, 14 einen Klettverschluss 15, 16 auf, der dadurch gebildet wird, dass ein Bereich 17 der Enden 13, 14 mit Widerhäkchen und ein Bereich 18 der Enden 13,14 mit Schlaufen versehen ist, was insbesondere in Figur 7 zu erkennen ist. Beim Umklappen der durch die jeweils zugeordneten Schlitze 9, 10 geführten Enden 13,14 gelangen die Bereiche 17 mit Widerhäkchen auf die Bereiche 18 mit Schlaufen, so die Enden 13, 14 jeweils eine Schlaufe 19, 20 bilden, die als Verschluss dient.

Zur Erzielung eines höheren Tragekomforts ist der Rückengurt 11 mit einer Polsterung 21 versehen, die aus elastischem Material gebildet wird.

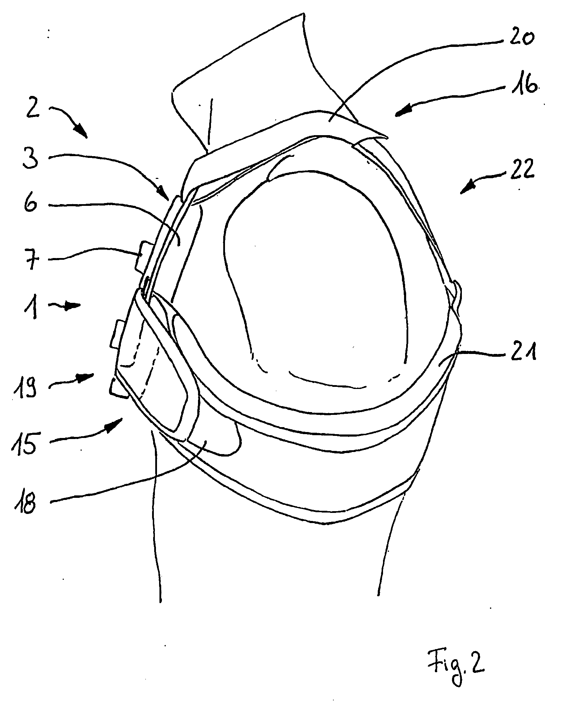

Die Figur 2 zeigt eine Seitenansicht des Patienten aus Figur 1, aus der die Verschnallung der Thorax-Bandage 2 besonders deutlich wird. Im Brustbereich 1 befindet sich die Trägerplatte 3 mit ihrem erhabenen Bereich 4, auf dessen Rückseite eine der Pelotten 6 mit ihren Verbindungszapfen 7 erkennbar ist.

Der Rückengurt 11 sowie die Schultergurte 12 sind mit ihren Enden 13, 14 durch die Schlitze 9, 10 in der Trägerplatte 3 geführt und bilden jeweils eine Schlaufe 19, 20.

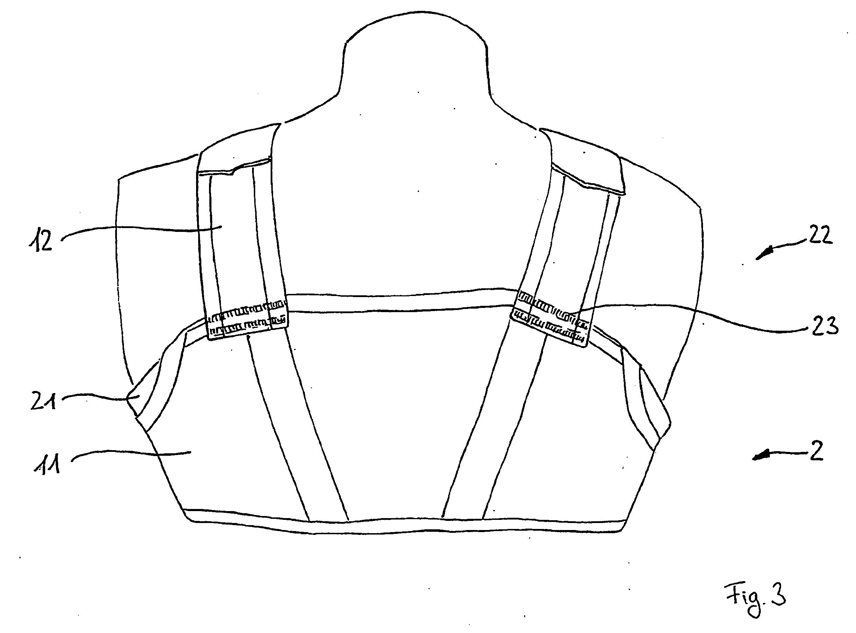

Im Rückenbereich 22 des Patienten sind die Schultergurte 12 mit dem Rückengurt 11 über Nähte 23 fest verbunden, was insbesondere aus der Figur 3 erkennbar ist.

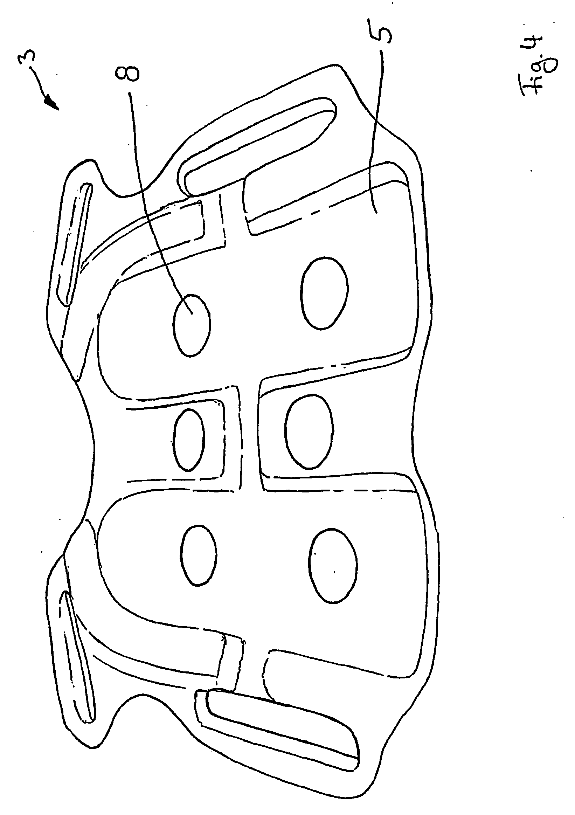

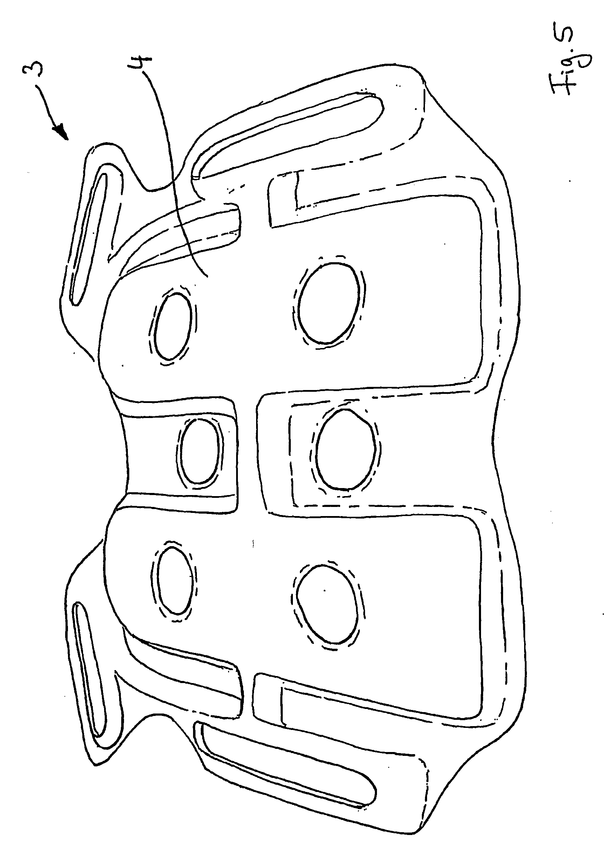

Die Figuren 4 und 5 zeigen die Trägerplatte 3 der erfindungsgemäßen Thorax-Bandage 2, die aus Polyethylen besteht und als Kunststoff-Tiefziehteil hergestellt ist, wobei sich Tiefziehen besonders zur Herstellung der Geometrie der Trägerplatte 3 mit ihren Mulden 5 und Durchbrüchen 8 eignet.

Bei der in der Figur 4 dargestellten Seite der Trägerplatte 3 handelt es sich um die Seite, die mit den Pelotten 6 versehen wird. Hierzu weist die Trägerplatte 3 den Pelotten 6 angepasste Mulden 5 auf, in die die Pelotten 6 eingesetzt werden. Zur Lagefixierung der Pelotten 6 ragen deren Verbindungszapfen 7 in die Durchbrüche 8 der Trägerplatte 3 hinein. Die Figur 5 zeigt die Trägerplatte 3 von der anderen Seite, auf der die den Mulden 5 korrespondierenden erhabenen Bereiche 4 zu erkennen sind.

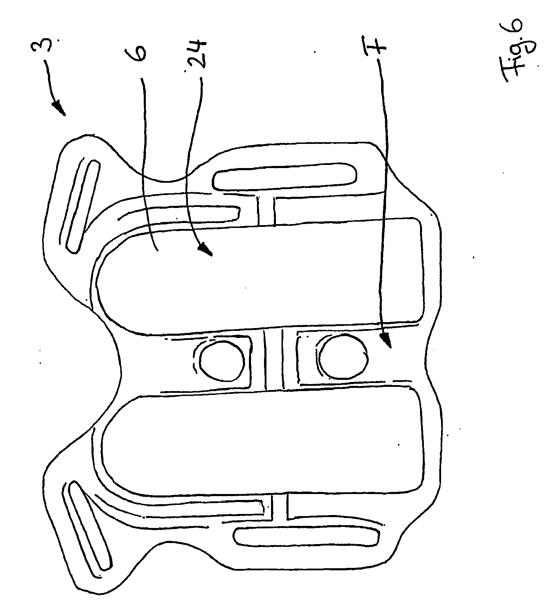

In der Figur 6 ist abermals die Trägerplatte 3 dargestellt, wobei diese nunmehr mit zwei Pelotten 6 ausgestattet ist. Die Pelotten 6 bestehen aus einem Schaumstoff und sind mit einem Baumwollstoff überzogen. Aus der Figur 6 wird die Geometrie und die Lage der Pelotten 6 zueinander deutlich. Die Pelotten 6 sind lang gestreckt und sind in einem Abstand und parallel zueinander auf der Trägerplatte 3 angeordnet, so dass sie einen Freiraum F im Bereich des Narbenschnitts des Patienten bilden. Die in der Figur 6 sichtbaren Oberflächen der beiden Pelotten bilden jeweils einen Druckbereich 24, mit dem die auf den Thorax auszuübende Kraft auf den Körper des Patienten übertragen wird.

Schließlich ist in der Figur 7 der Rückengurt 11 mit den daran befestigten Schultergurten 12 abgebildet, wobei die Enden 13, 14 von dem Rückengurt 11 und den Schultergurten 12 mit ihren Klettverschlüssen 15, 16 nichtals Schlaufe 19,20 umgeklappt sind. Auf diese Weise sind die Bereiche 17 mit Widerhäkchen und die Bereiche mit Schlaufen, die jeweils einen Klettverschluss 15, 16 bilden, gut erkennbar.

Um den Tragekomfort der erfindungsgemäßen Thorax-Bandage 2 weiter zu erhöhen, können der Rückgurt 11 und die Schultergurte 12 ferner Abschnitte aus einem elastisch dehnbaren Material aufweisen.

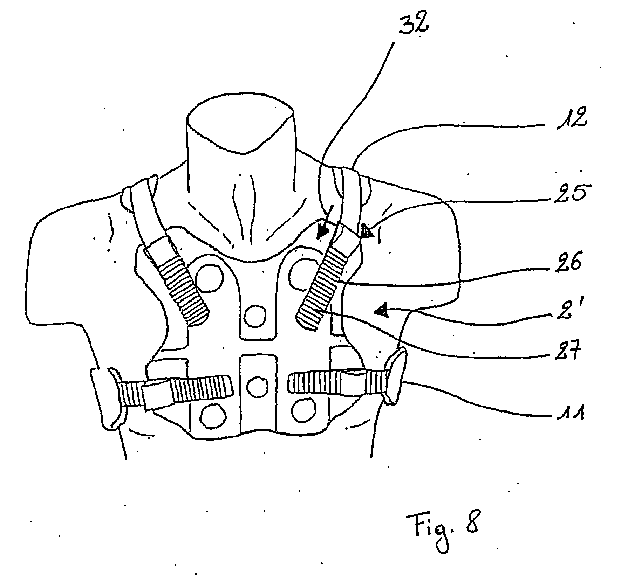

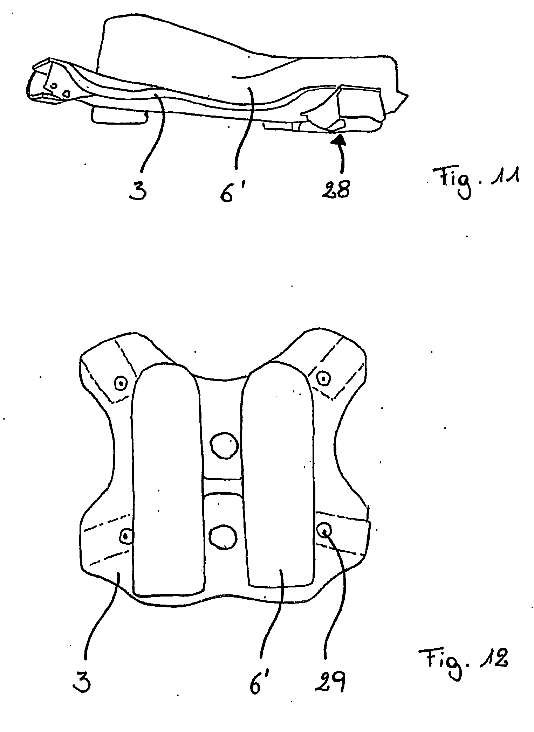

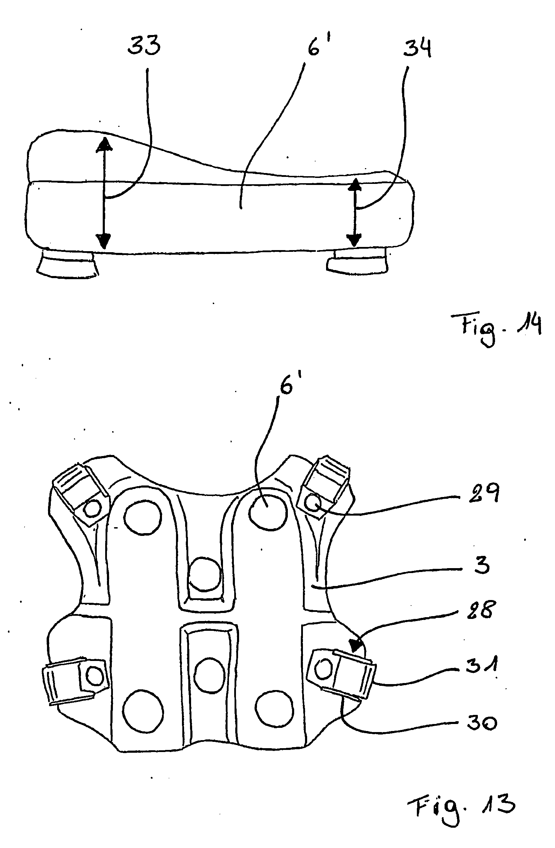

Eine alternative Ausführungsform einer Thorax-Bandage 2' ist in den Figuren 8 bis 14 in verschiedenen Ansichten dargestellt. Wie sich aus Figur 8 entnehmen lässt, erfolgt die Fixierung der Thorax-Bandage 2' am Patienten, d.h. die Gurtfixierung, mit Hilfe so genannter Rasterverschlüsse 25, die jeweils an den gegenüber liegenden Enden des zirkulären Rückengurts 11 sowie den jeweiligen Enden der beiden Schultergurte 12 angeordnetsind. Wie sich insbesondere aus den Figuren 8 und 13 ergibt, besteht jeder Rasterverschluss 25 aus einer an den Enden der Gurte befestigten Zunge 26, die an ihrer dem Patienten abgewandten Oberseite mit einer widerhakenförmigen Verzahnung 27 versehen ist. Die Zungen 26 wirken jeweils mit einer von insgesamt vier Schließköpfen 28 zusammen, die mit Hilfe von Edelstahlnieten 29an der Trägerplatte befestigt sind. Jeder Schließkopf 28 besteht aus einem Grundkörper 30, der zwei aufgekantete Wangen besitzt, an denen drehbar gelagert jeweils eine Sperrklinke 31 befestigt ist. Aufgrund einer Federvorspannung liegt die Sperrklinke 31 mit einem nicht sichtbaren Sperrabschnitt an der mit der Verzahnung 27 versehenen Oberfläche der Zungen 26 an und greift rastend jeweils in den Freiraum zwischen zwei benachbarten Zähnen. Während ein Einführen der Zungen 26 in Richtung des Pfeils 32 ebenso möglich ist wie ein weiteres Durchschieben der Zunge 26 unter der Sperrklinke 30 hindurch, ist ein rückwärts gerichtetes Herausziehen erst nach Lösen der Sperrklinke 31 durch Druck auf dieselbe möglich, wodurch das Sperrelement aus der Verzahnung 27 herausgehoben wird.

Mit Hilfe der vorstehend erläuterten Rasterverschlüsse 25 lässt sich eine sehr feinfühlige und für den Patienten einfach und schmerzfrei zu bewerkstelligende Einstellung der Anpresskraft der Thorax-Bandage 2' erzielen.

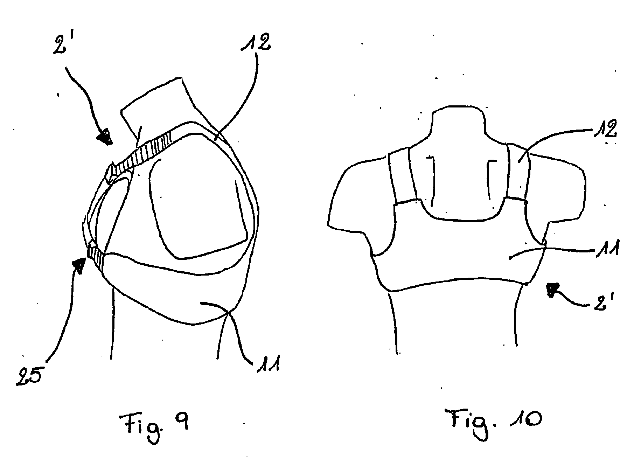

Aus den Figuren 10 und 14 lässt sich schließlich noch entnehmen, dass die Pelotte 6' auch eine in Längsrichtung des Sternums veränderliche Dicke aufweisen kann. Um der Anatomie des Thorax besser gerecht zu werden und auch im oberen Bereich des Sternums, der dem Hals zugewandt ist, einen hinreichend großen Anpressdruck zu erzielen, ist die Dicke 33 der Pelotte dort ca. 30 % bis 50 % größer als die Dicke 34 am unteren Ende der Pelotte 6'. Der Übergang zwischen den Bereichen mit unterschiedlicher Dicke erfolgt der Anatomie des Menschen angepasst fließend und wellenförmig.

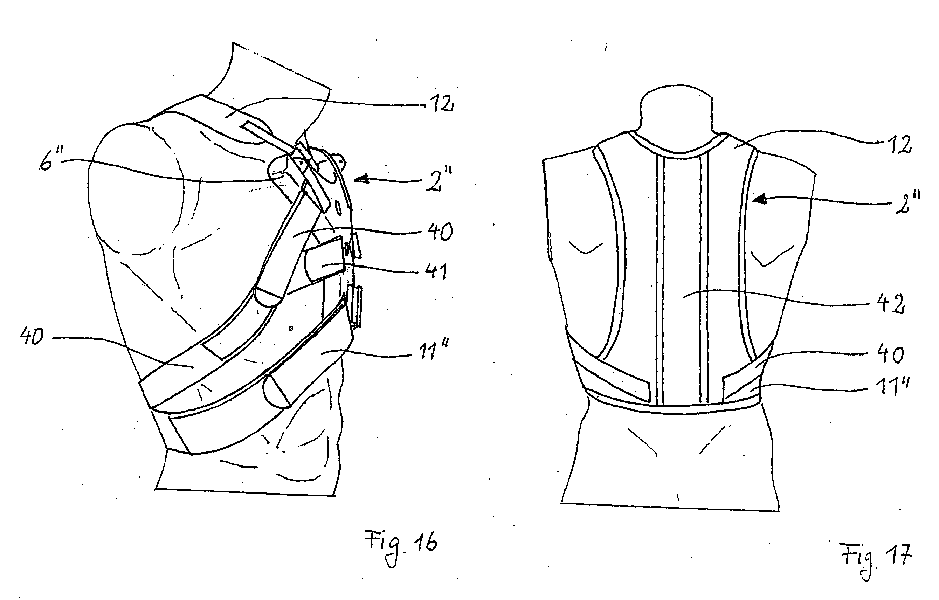

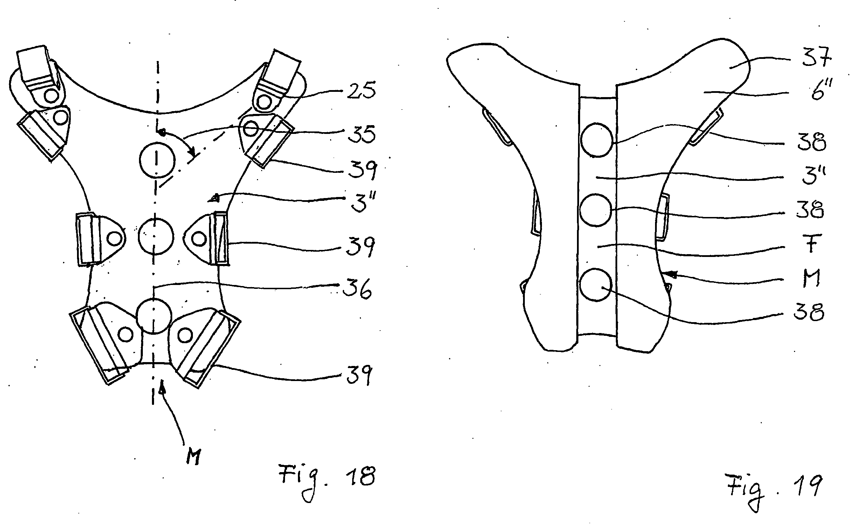

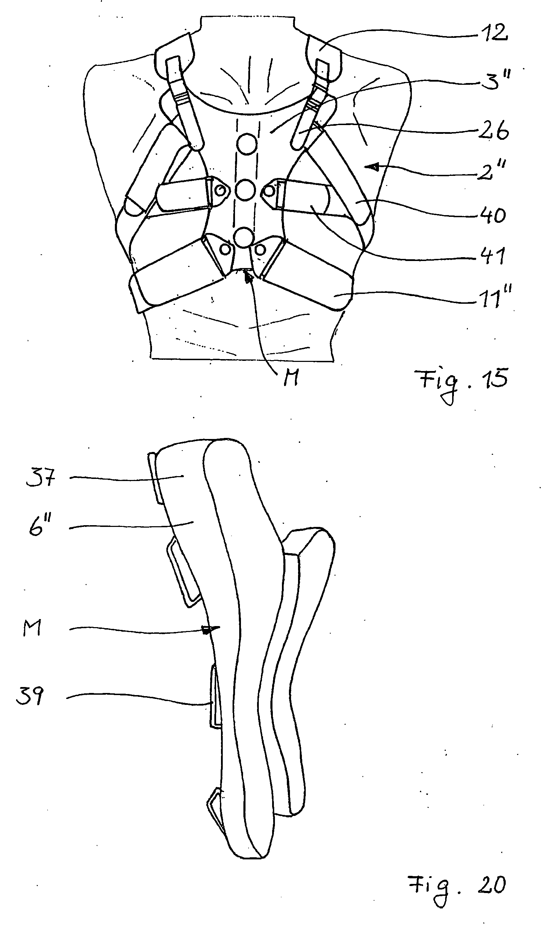

Eine dritte Ausführungsform einerThorax-Bandage 2" ist in den Figuren 15 bis 20 dargestellt. Die das Mittelteil M bildende Trägerplatte 3" besitzt in diesem Fall (s. Fig. 18) eine ungefähr Y-förmige Gestalt. Die Trägerplatte 3" weist zwei unter einem Winkel 35 von ca. 45° zu einer Mittellängsachse 36 der Trägerplatte 3" ausgehende armförmige Fortsätze 37 auf. Wie sich insbesondere aus den Seitenansichten in den Figuren 16 und 20 ergibt, ist die Trägerplatte 3" einschließlich der Fortsätze 37 gewölbt, um eine gute Anpassung im Brustkorbbereich des Patienten zu erhalten.

Wie sich insbesondere aus Figur 19 ergibt, ist die Trägerplatte 3" auf der den Patienten zugewandten Seite mit zwei spiegelbildlich zueinander angeordneten Pelotten 6" aus einem Schaumstoffmaterial versehen (und zwar flächig verklebt), die sich auch im Bereich der Fortsätze 37 befinden. Zwischen den gegenüberliegenden Pelotten 6" befindet sich ein Freiraum F, der oberhalb einer Wunde im Bereich des Sternums des Patienten angeordnet ist. Die Trägerplatte 3" ist im Bereich zwischen den Pelotten 6" mit drei in einer linearen Reihe angeordneten Durchbrüchen 38 mit jeweils kreisförmiger Kontur versehen. Wie sich insbesondere aus Figur 15 entnehmen lässt, kann durch diese Durchbrüche 38 hindurch eine visuelle Kontrolle der Wundheilung im Bereich des Sternums durchgeführt werden, ohne die Thorax-Bandage 2" entfernen zu müssen.

Der Figur 18 lässt sich entnehmen, dass die Trägerplatte 3" auf der Vorderseite mit insgesamt sechs Ösen 39 versehen ist, die eine flach rechteckförmige Gestalt aufweisen und zur Durchführung dreier zirkulärer Rückengurte 11", 40, 41 (bzw. von deren Schlaufen) dienen, wobei die Befestigung der Rückengurte 11", 40, 41 jeweils mit an den Enden angeordneten Klettverschlüssen erfolgt. Die beiden Rückengurte 40, 41 laufen in relativ geringem Abstand von der Trägerplatte 3" zusammen.

Gemäß Figur 18 ist an den Fortsätzen 37 jeweils ein Rasterverschluss 25 befestigt, der jeweils mit einer verzahnten Zunge 26 eines Schultergurts 12 zusammenwirkt (Figur 15).

Aus der Figur 17 lässt sich entnehmen, dass die Rückengurte 11" und 40 an einem flächigen Rückenteil 42 aus textilem Material enden. Während die Rükkengurte 11" und 40 an der Unterseite des Rückenteils 42 anschließen, gehen die Schultergurte 12 von dessen Oberseite aus.

Wie sich schließlich noch aus Figur 20 entnehmen lässt, ist die Dicke der Pelotte 6" im Bereich einer mittleren Öse 39 für den Rückengurt 41 am geringsten und nimmt von dort aus sowohl nach unten als auch zu den Fortsätzen 37 hin zu, wobei sie im Bereich der Fortsätze 37 maximal ist. Diese Gestalt kommt der menschlichen Physiognomie am nächsten und sichert ein möglichst beschwerdefreies Tragen der Thorax-Bandage 2".

### Bezugszeichenliste:

- 1: Brustbereich
- 2, 2', 2": Thorax-Bandage
- 3, 3": Trägerplatte
- 4: Erhabener Bereich
- 5: Mulde
- 6, 6', 6": Pelotte
- 7: Verbindungszapfen
- 8: Durchbruch
- 9: Schlitz
- 10: Schlitz
- 11: Rückengurt
- 12: Schultergurt
- 13: Ende
- 14: Ende
- 15: Klettverschluss
- 16: Klettverschluss
- 17: Bereich mit Widerhäkchen
- 18: Bereich mit Schlaufen
- 19: Schlaufe
- 20: Schlaufe
- 21: Polsterung
- 22: Rückenbereich
- 23: Naht
- 24: Druckbereich
- 25: Rasterverschluss
- 26: Zunge
- 27: Verzahnung
- 28: Schließkopf
- 29: Edelstahl-Niet
- 30: Grundkörper
- 31: Sperrklinke
- 32: Pfeil
- 33: Dicke
- 34: Dicke
- 35: Winkel
- 36: Mittellängsachse
- 37: Fortsatz
- 38: Durchbruch
- 39: Öse
- 40: Rückengurt
- 41: Rückengurt
- 42: Rückenteil
- F: Freiraum
- M: Mittelteil

## Patentansprüche

1. Thorax-Bandage (2, 2', 2") mit einem mindestens eine Pelotte (6, 6', 6") aus einem elastischen Material, insbesondere einem Schaumstoff, aufweisenden Mitteilteil (M), an dessen gegenüberliegenden horizontalen Enden jeweils die Enden (13) eines zirkulären Rückengurts (11) angeschlossen sind und an dessen oberem Ende beabstandet voneinander die Enden (14) zweier Schultergurte (12) angeschlossen sind, wobei beide Schuitergurte (12) im Rückenbereich (22) eines Patienten mit dem Rückgurt (11) verbunden sind, **dadurch gekennzeichnet, dass** das Mittelteil (M) eine Trägerplatte (3) aufweist,
- an der die mindestens eine Pelotte (6, 6', 6") befestigt ist,
- die aus einem im Vergleich mit dem Material der Pelotte (6, 6') wesentlich steiferen Material besteht,
- an die der Rückengurt (11) und die Schultergurte (12) angeschlossen sind,
- die den Bereich des Sternums eines Patienten starr überbrückt, wobei
- die mindestens eine Pelotte (6, 6', 6") zwei lang gestreckte, parallel zu dem Sternum des Patienten verlaufende und seitlich neben diesem am Thorax anliegende Druckbereiche aufweist, die von einer rinnenförmigen und parallel zu den Druckbereichen verlaufenden Vertiefung im Bereich des Sternum voneinander getrennt sind.

2. Thorax-Bandage nach Anspruch 1, **gekennzeichnet durch** zwei lang gestreckte Pelotten (6, 6', 6"), die parallel zu dem Sternum des Patienten verlaufen und seitlich neben diesem am Thorax des Patienten anliegen.

3. Thorax-Bandage nach Anspruch 2, **gekennzeichnet durch** einen langgestreckten Freiraum (F) zwischen den beiden Pelotten (6, 6', 6") bzw. den beiden Druckbereichen (24) einer Pelotte (6, 6', 6"), wobei der Frelraum (F) im Querschnitt vorzugsweise eine Breite zwischen 3 cm und 7 cm und eine Höhe zwischen 2 cm und 5 cm aufweist.

4. Thorax-Bandage nach Anspruch 3, **dadurch gekennzeichnet, dass** die Trägerplatte (3, 3") im Bereich des Freiraums (F) mit mindestens einem Durchbruch (38), vorzugsweise einer Reihe hintereinander angeordneter Durchbrüche (38), versehen ist, durch den bzw. die hindurch eine Wunde im Bereich des Sternums des Patienten visuell kontrollierbar ist.

5. Thorax-Bandage nach einem der Ansprüche 1 bis 4, **dadurch gekennzeichnet, dass** die mindestens eine Pelotte (6, 6') lösbar mit der Trägerplatte (3) verbunden ist, insbesondere mittels mindestens eines einstückig mit der Pelotte (6, 6') verbundenen Verbindungszapfens (7) formschlüssig und/oder reibschlüssig in efnen angepassten Durchbruch (8) in der Trägerplatte (3) eingreift.

6. Thorax-Bandage nach einem der Ansprüche 1 bis 5, **dadurch gekennzeichnet, dass** die Enden (13, 14) des Rückengurtes (11) und/oder der Schultergurte (12) in Form von Schlaufen (19, 20) durch randnah angeordnete Schlitze (9, 10) in der Trägerplatte (3) oder durch flach-rechteckförmige an der Trägerplatte (3") befestigte Ösen (39) geführt sind.

7. Thorax-Bandage nach einem der Ansprüche 1 bis 6, **dadurch gekennzeichnet, dass** der Rückengurt (11) und/oder die Schultergurte (12) mit mindestens einem Abschnitt aus einem in ihre Längsrichtung elastisch dehnbarem Material versehen sind.

8. Thorax-Bandage nach einem der vorgenannten Ansprüche, **dadurch gekennzeichnet, dass** die Schlaufen (19, 20) des Rückengurts (11) und/oder der Schultergurte (12) mit Klettverschlüssen (15, 16) versehen sind, die eine Längeneinstellung des Rückengurtes (11) und/oder der Schultergurte (12) erlauben.

9. Thorax-Bandage nach einem der Ansprüche 1 bis 7, **dadurch gekennzeichnet, dass** die wirksame Länge des Rückengurts (11) und/oder der Schultergurte (12) jeweils mittels eines Rasterverschlusses (25) einstellbar ist, wobei mit widerhakenförmigen Zähnen versehene Zungen (26) an den Enden des Rückengurtes (11) und/oder der Schultergurte (12) rastend in verschiedenen Positionen in zugeordneten Schließköpfen (28) der Trägerplatte (3) arretierbar sind.

10. Thorax-Bandage nach einem der Ansprüche 1 bis 9, **dadurch gekennzeichnet, dass** der zirkuläre Rückengurt (11) im Bereich der Achsel des Patienten mit einem Ausschnitt und einer entlang des Randes des Ausschnitts verlaufenden Polsterung (24) aus einem elastischen Material versehen ist.

11. Thorax-Bandage nach einem der Ansprüche 1 bis 10, **dadurch gekennzeichnet, dass** die aus einem Kunststoff-Schaummaterial bestehenden Pelotten (6) eine zum Hals des Patienten hin zunehmende Dicke besitzen.

12. Thorax-Bandage nach einem der Ansprüche 1 bis 11, **dadurch gekennzeichnet, dass** die Trägerplatte (3,3") mit zwei armförmigen Fortsätzen (37) versehen ist, die jeweils in Richtung auf eine Schulter des Patienten zu erstrecken, wodurch die Trägerplatte (3") eine Y-förmige Gestalt erhält, wobei die Schultergurte (12) an die freien Enden der Fortsätze (37) anschließen.

13. Thorax-Bandage nach einem der Ansprüche 1 bis 12, **dadurch gekennzeichnet, dass** der Rückengurt (11) und die Schultergurte (12) von einem auf dem Rücken des Patienten angeordneten flächigen Rückteil (42) ausgehen, das vorzugsweise eine Breite von mindestens 20 cm und eine Länge von mindestens 40 cm besitzt, wobei der Rückengurt (11) an die Unterseite des Rückenteils (42) und die Schultergurte (12) an die Oberseite des Rückteils (42) anschließen.

## Claims

1. A thorax bandage (2, 2', 2") with a central part (M) having at least one pelotte (6, 6', 6") of an elastic material, in particular a foam, at the opposite horizontal ends of which respectively the ends (13) of a circular back strap (11) are connected and at the upper end of which the ends (14) of two shoulder straps (12) are connected, spaced apart from one another, wherein both shoulder straps (12) are connected with the back strap (11) in the back area (22) of a patient, **characterized in that** the central part (M) has a support plate (3),
- on which the at least one pelotte (6, 6', 6") is fastened,
- which consists of a substantially stiffer material compared with the material of the pelotte (6, 6'),
- to which the back strap (11) and the shoulder straps (12) are connected,
- which bridges in a rigid manner the region of the sternum of a patient, wherein
- the at least one pelotte (6, 6', 6") has two elongate pressure regions running parallel to the sternum of the patient and lying laterally adjacent thereto on the thorax, which pressure regions are separated from one another by a channel-shaped depression running parallel to the pressure regions in the region of the sternum.

2. The thorax bandage according to Claim 1, **characterized by** two elongate pelottes (6, 6', 6") which run parallel to the sternum of the patient and lie laterally adjacent thereto on the thorax of the patient.

3. The thorax bandage according to Claim 2, **characterized by** an elongate free space (F) between the two pelottes (6, 6', 6") or respectively the two pressure regions (24) of a pelotte (6, 6', 6"), wherein the free space (F) in cross-section preferably has a width of between 3 cm and 7 cm and a height of between 2 cm and 5 cm.

4. The thorax bandage according to Claim 3, **characterized in that** the support plate (3, 3") is provided in the region of the free space (F) with at least one aperture (38), preferably a series of apertures (38) arranged one behind another, through which a wound in the region of the sternum of the patient is able to be visually monitored.

5. The thorax bandage according to one of Claims 1 to 4, **characterized in that** the at least one pelotte (6, 6') is detachably connected with the support plate (3), in particular engages in a form-fitting and/or friction-fitting manner into an adapted aperture (8) in the support plate (3) by means of at least one connecting pin (7) which is connected in one piece with the pelotte (6, 6').

6. The thorax bandage according to one of Claims 1 to 5, **characterized in that** the ends (13, 14) of the back strap (11) and/or of the shoulder straps (12) in the form of loops (19, 20) are guided through slits (9, 10), arranged close to the edge, in the support plate (3) or through flat rectangle-shaped eyes (39) fastened to the support plate (3").

7. The thorax bandage according to one of Claims 1 to 6, **characterized in that** the back strap (11) and/or the shoulder straps (12) are provided with at least one section of a material which is able to be stretched elastically in the longitudinal direction thereof.

8. The thorax bandage according to one of the preceding claims, **characterized in that** the loops (19, 20) of the back strap (11) and/or of the shoulder straps (12) are provided with hook-and-pile fasteners (15, 16), which permit a length adjustment of the back strap (11) and/or of the shoulder straps (12).

9. The thorax bandage according to one of Claims 1 to 7, **characterized in that** the effective length of the back strap (11) and/or of the shoulder straps (12) is adjustable respectively by means of a detent closure (25), wherein tongues (26) provided with barb-shaped teeth at the ends of the back strap (11) and/or of the shoulder straps (12) are able to be held in place in an arresting manner in various positions in associated closure heads (28) of the support plate (3).

10. The thorax bandage according to one of Claims 1 to 9, **characterized in that** the circular back strap (11) is provided in the region of the armpits of the patient with a cut-out and with a padding (24) of an elastic material running along the edge of the cut-out.

11. The thorax bandage according to one of Claims 1 to 10, **characterized in that** the pelottes (6), consisting of a plastic foam material, have a thickness which increases towards the neck of the patient.

12. The thorax bandage according to one of Claims 1 to 11, **characterized in that** the support plate (3, 3") is provided with two arm-shaped extensions (37) which extend respectively towards a shoulder of the patient, whereby the support plate (3") is given a Y-shaped configuration, wherein the shoulder straps (12) adjoin the free ends of the extensions (37).

13. The thorax bandage according to one of Claims 1 to 12, **characterized in that** the back strap (11) and the shoulder straps (12) proceed from a flat back part (42) arranged on the patient's back, which back part preferably has a width of at least 20 cm and a length of at least 40 cm, wherein the back strap (11) adjoins the underside of the back part (42) and the shoulder straps (12) adjoin the upper side of the back part (42).

## Revendications

1. Bandage de thorax (2, 2', 2") doté d'au moins une pelote (6, 6', 6") faite d'une pièce centrale (M) présentant un matériau élastique, en particulier une mousse, aux extrémités horizontales opposées de laquelle sont raccordées respectivement les extrémités (13) d'une sangle dorsale circulaire (11) et à l'extrémité supérieure de laquelle sont raccordées à distance l'une de l'autre les extrémités (14) de deux sangles d'épaule (12), les deux sangles d'épaule (12) étant reliées au niveau du dos (22) d'un patient à la sangle dorsale (11), **caractérisé en ce que** la pièce centrale (M) présente une plaque support (3)
- à laquelle l'au moins une pelote (6, 6', 6") est fixée,
- qui est composée d'un matériau plus rigide que le matériau de la pelote (6, 6'),
- à laquelle la sangle dorsale (11) et les sangles d'épaule (12) sont reliées,
- qui chevauche avec rigidité la zone du sternum d'un patient,
- l'au moins une pelote (6, 6', 6") présentant deux zones de compression étirées en longueur s'étendant parallèlement au sternum du patient et en contact latéral avec celui-ci par son thorax et qui sont séparées l'une de l'autre par un creux en forme de rigole s'étendant parallèlement aux zones de compression au niveau du sternum.

2. Bandage de thorax selon la revendication 1, **caractérisé par** deux pelotes étirées en longueur (6, 6', 6") qui s'étendent parallèlement au sternum du patient et sont en contact latéral avec celui-ci par son thorax.

3. Bandage de thorax selon la revendication 2, **caractérisé par** un espace libre étiré en longueur (F) entre les deux pelotes (6, 6', 6") ou les deux zones de compression (24) d'une pelote (6, 6', 6"), l'espace libre (F) présentant en section transversale de préférence une largeur comprise entre 3 cm et 7 cm et une hauteur comprise entre 2 cm et 5 cm.

4. Bandage de thorax selon la revendication 3, **caractérisé en ce que** la plaque support (3, 3") est pourvue au niveau de l'espace libre (F) d'au moins une percée (38), de préférence d'une série de percées se succédant (38), à travers laquelle ou lesquelles une blessure située au niveau du sternum du patient est visuellement contrôlable.

5. Bandage de thorax selon une des revendications 1 à 4, **caractérisé en ce que** l'au moins une pelote (6, 6') est reliée de manière dissociable à la plaque support (3), en particulier s'engrène en correspondance géométrique et/ou de friction dans une percée adaptée (8) de la plaque support (3) au moyen d'au moins un tourillon de raccordement (7) relié à la pelote (6, 6') en formant une seule pièce.

6. Bandage de thorax selon une des revendications 1 à 5, **caractérisé en ce que** les extrémités (13, 14) de la sangle dorsale (11) et/ou des sangles d'épaule (12) passent sous forme de boucles (19, 20) dans des fentes (9, 10) pratiquées près du bord de la plaque support (3) ou dans des oeillets (39) de forme plate et rectangulaire de la plaque support (3").

7. Bandage de thorax selon une des revendications 1 à 6, **caractérisé en ce que** la sangle dorsale (11) et/ou les sangles d'épaule (12) sont pourvues d'au moins une découpe en matériau extensible élastiquement dans son sens longitudinal.

8. Bandage de thorax selon une des revendications précédentes, **caractérisé en ce que** les boucles (19, 20) de la sangle dorsale (11) et/ou des sangles d'épaule (12) sont pourvues de fermetures agrippantes (15, 16) qui permettent un réglage en longueur de la sangle dorsale (11) et/ou des sangles d'épaule (12).

9. Bandage de thorax selon une des revendications 1 à 7, **caractérisé en ce que** la longueur utile de la sangle dorsale (11) et/ou des sangles d'épaule (12) est réglable respectivement au moyen d'une fermeture à enclenchement (25), des languettes (26) pourvues de dents en forme d'ardillons pouvant être bloquées aux extrémités de la sangle dorsale (11) et/ou des sangles d'épaule (12) en s'enclenchant dans différentes positions dans des têtes de fermeture associées (28) de la plaque support (3).

10. Bandage de thorax selon une des revendications 1 à 9, **caractérisé en ce que** la sangle dorsale circulaire (11) est pourvue au niveau de l'aisselle du patient d'une échancrure et d'un rembourrage (24) en matériau élastique s'étendant le long du bord de l'échancrure.

11. Bandage de thorax selon une des revendications 1 à 10, **caractérisé en ce que** les pelotes (6) faites d'un matériau de mousse plastique ont une épaisseur croissante en direction du cou du patient.

12. Bandage de thorax selon une des revendications 1 à 11, **caractérisé en ce que** la plaque support (3, 3") est pourvue de deux prolongements en forme de bras (37) qui s'étendent respectivement en direction d'une épaule du patient, ce qui donne à la plaque support (3") une forme de Y, les sangles d'épaule (12) se raccordant aux extrémités libres des prolongements (37).

13. Bandage de thorax selon une des revendications 1 à 12, **caractérisé en ce que** la sangle dorsale (11) et/ou les sangles d'épaule (12) partent d'une pièce dorsale plane (42) disposée sur le dos du patient et qui a de préférence une largeur d'au moins 20 cm et une longueur d'au moins 40 cm, la sangle dorsale (11) se raccordant à la partie inférieure de la pièce dorsale (42) et la sangle d'épaule (12) à la face supérieure de la pièce dorsale (42).
